# EUROPEAN PATENT SPECIFICATION

(11) **EP 3 088 043 B1**
(45) Date of publication and mention of the grant of the patent: **03.01.2018**
(21) Application number: 16160049.9
(22) Date of filing: 13.03.2016
(51) Int. Cl.: A61N 1/32, A61H 23/02, A61H 39/04, A61F 9/04

(54) **MASSAGE DEVICE FOR ALLEVIATING DRIVING FATIGUE**
MASSAGE VORRICHTUNG, UM FAHREN ERMÜDUNG ZU LINDERN
DISPOSITIF DE MASSAGE POUR SOULAGER LA FATIGUE DE CONDUIRE

(30) Priority: 29.04.2015 CN 201510210753
(43) Date of publication of application: 02.11.2016
(73) Proprietor: Protruly Vision Technology Group Co., Ltd., Nanjing Jiangsu 210000 (CN)
(72) Inventor: LONG, Gang, Nanjing Jiangsu 210000 (CN); LIN, Songwei, Nanjing Jiangsu 210000 (CN)
(74) Representative: Dargiewicz, Joanna

(56) References cited:
- CN-A- 101 156 814
- CN-A- 104 337 614
- CN-U- 202 086 751
- CN-U- 202 557 300
- CN-U- 202 909 085
- CN-Y- 2 524 721
- CN-Y- 200 963 235
- CN-Y- 201 179 144
- US-A1- 2011 234 971

## Description

### TECHNICAL FIELD

The present invention relates to the technical field of prevention of fatigue driving, in particular to an anti-fatigue driving massage device.

### BACKGROUND OF THE PRESENT INVENTION

The currently existing anti-fatigue driving technologies are eyeball tracking, head track, human face recognition, etc. which perform change monitoring based on the features of videos or an electrocardiogram, pulse, an electroencephalogram, etc. These anti-fatigue driving technologies have the following disadvantages: firstly, the recognition rate is not high, and the false detection rate and the missing detection rate are high; and secondly, when fatigue driving is recognized, a tragedy often has happened. Documents with patents No. CN-Y-200963235, CN-A-104337614, CN-U-202557300, CN-U-202909085 and CN-A-101156814 have disclosed some massage devices. Most of them massage by vibration, and some massage by micro-current. However, the vibrator and massage head are generally mounted together in the massager. Most of them are applied in eyeglasses and the application is limited.

### SUMMARY OF THE PRESENT INVENTION

The invention is defined in claim 1. A further aspect is defined in the appended claim. Aspects, embodiments and examples of the present disclosure which do not fall under the scope of the appended claims do not form part of the invention and are merely provided for illustrative purposes. Furthermore, the methods presented in the present description are provided for illustrative purposes only and do not form part of the present invention.

The purpose of the present invention is to provide an anti-fatigue driving massage device convenient in usage and capable of performing automatic timing control, so as to solve the problems proposed in the above-mentioned background.

To achieve the above-mentioned purpose, the present invention provides the following technical solution:
An anti-fatigue driving massage device comprises a control system, a control device, a fixing device, a magnetic film, a power supply and a man-machine interactive device, wherein the control system is respectively connected with the control device, the power supply and the man-machine interactive device; the control device is fixedly installed on the fixing device; and the magnetic film is stuck to the muscle around an eye of a driver.

As a further solution of the present invention: the control device is a loop current control device with a coil or a control device with a motor and magnets.

As a further solution of the present invention: the fixing device is a spectacle frame or a sun shield.

As another further solution of the present invention: the power supply is a battery or a vehicular electric power supply.

The usage method of the anti-fatigue driving massage device comprises the specific steps as follows:
1) installing the control system and the power supply, fixedly installing the control device on the spectacle frame or the sun shield, and sticking the magnetic film to the muscle around the eye of the driver;
2) setting or regulating the massage strength and period through the man-machine interactive device;
3) obtaining a parameter of a current direction, a current size or the rotational speed of the motor matched with the present massage strength and period by the control system through corresponding algorithm processing; and
4) alternately controlling the polarity of a magnetic field around the control device by the control system through the parameter obtained in step 3), thereby controlling the magnetic film to massage the muscle around the eye.

Compared with the prior art, the present invention has the beneficial effects that:
In the present invention, the control system is used for automatically controlling the magnetic film at regular time to perform active anti-fatigue massage, stimulus and the like for alerting the driver and relieving the fatigue of the driver, so that the driver keeps a conscious driving state; and the driver does not need to perform any operation during driving, thereby improving the driving safety.

### BRIEF DESCRIPTION OF THE DRAWINGS

Fig. 1 is a structural diagram of the present invention.
Fig. 2 is a realization flow chart of the present invention.
Fig. 3 is a position diagram of a control device and a fixing device in embodiment 1.
Fig. 4 is a position diagram of a control device and a fixing device in embodiment 2.
Fig. 5 is a position diagram of a control device and a fixing device in embodiment 3.

In the figures: 1-control system; 2-control device; 3-fixing device; 4-magnetic film; 5-power supply; 6-man-machine interactive device; 7-magnet; and 8-motor.

### DETAILED DESCRIPTION OF THE PRESENT INVENTION

The technical solution of the present patent will be further described in detail as below with the combination of the specific embodiments.

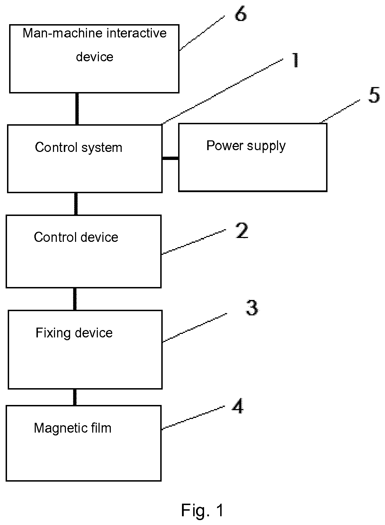

Please with reference to Fig. 1 to Fig. 2, an anti-fatigue driving massage device comprises a control system 1, a control device 2, a fixing device 3, a magnetic film 4, a power supply 5 and a man-machine interactive device 6, wherein the power supply 5 is a battery or a vehicular electric power supply; the control system 1 is respectively connected with the control device 2, the power supply 5 and the man-machine interactive device 6; the control device 2 is fixedly installed on the fixing device 3; the fixing device 3 is a spectacle frame or a sun shield; the magnetic film 4 is stuck to the muscle around an eye of a driver; and the control device 2 is a loop current control device with a coil or a control device with a motor 8 and magnets 7. The control device with the motor 8 and the magnets 7 changes the polarity of the magnets 7 through the rotation of the motor 8; the control system 1 uses an electromagnetic induction law to control the size of the current of the coil so as to control the intensity of a magnetic field around a loop current control device with the coil, and to control the direction of the current so as to control the polarity of the magnetic field around the loop current control device with the coil; and the control system 1 uses the characteristic of the magnets 7 that like poles repel and unlike poles attract to alternately control the polarity of the magnetic field around the control device with the motor 8 and the magnets 7, so as to control the magnetic film 4 to perform massage actions of inward pressing, outward pull, etc. on the muscle around the eye, thereby achieving the effects of relieving the fatigue and alerting safe driving.

The usage method of the anti-fatigue driving massage device comprises the specific steps as follows:
1) installing the control system 1 and the power supply 5, fixedly installing the control device 2 on the spectacle frame or the sun shield, and sticking the magnetic film 4 to the muscle around the eye of the driver, wherein the magnetic film 4 is required not to shield the sight line of the driver;
2) setting or regulating the massage strength and period through the man-machine interactive device 6;
3) obtaining a parameter of a current direction, a current size or the rotational speed of the motor 8 matched with the present massage strength and period by the control system 1 through corresponding algorithm processing; and
4) alternately controlling the polarity of the magnetic field around the control device 2 by the control system 1 through the parameter obtained in step 3), thereby controlling the magnetic film 4 to massage the muscle around the eye.

### Embodiment 1

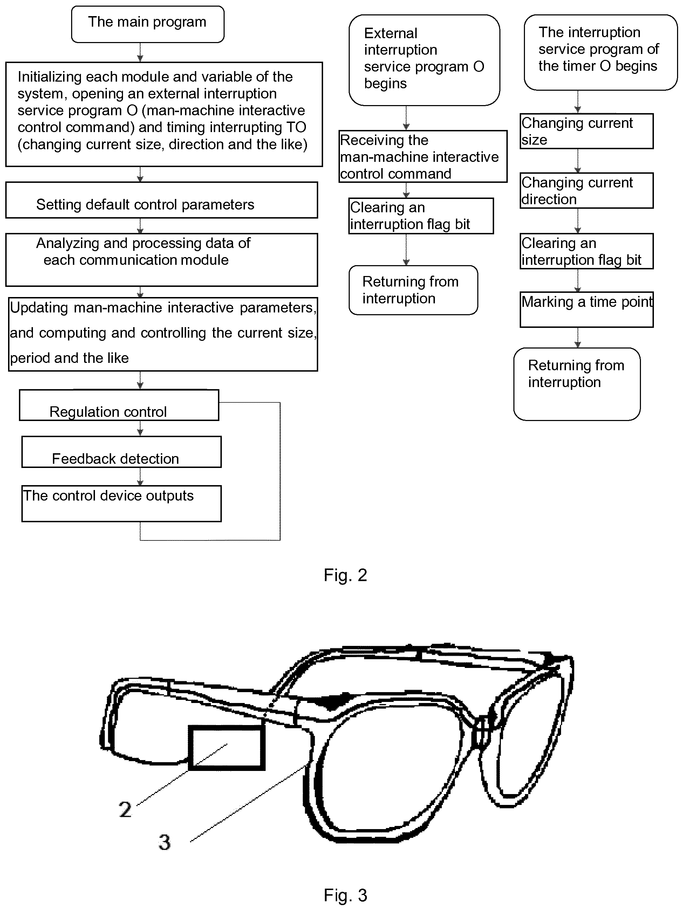

Please with reference to Fig. 3, in the embodiment of the present invention, an anti-fatigue driving massage device comprises a control system 1, a control device 2, a fixing device 3, a magnetic film 4, a power supply 5 and a man-machine interactive device 6, wherein the power supply 5 is a battery; the control system 1 is respectively connected with the control device 2, the power supply 5 and the man-machine interactive device 6; the control device 2 is fixedly installed on the fixing device 3; the fixing device 3 is a spectacle frame; and the control device 2 is a loop current control device with a coil.

The usage method of the anti-fatigue driving massage device comprises the specific steps as follows:
1) installing the control system 1 and the power supply 5, fixedly installing the loop current control device with the coil on the spectacle frame, and sticking the magnetic film 4 to the muscle around the eye of the driver, wherein the magnetic film 4 is required not to shield the sight line of the driver;
2) setting or regulating the massage strength and period through the man-machine interactive device 6;
3) obtaining the parameters of a current direction and a current size matched with the present massage strength and period by the control system 1 through corresponding algorithm processing; and
4) alternately controlling the polarity of the magnetic field around the loop current control device with the coil by the control system 1 through the parameters obtained in step 3), thereby controlling the magnetic film 4 to massage the muscle around the eye.

### Embodiment 2

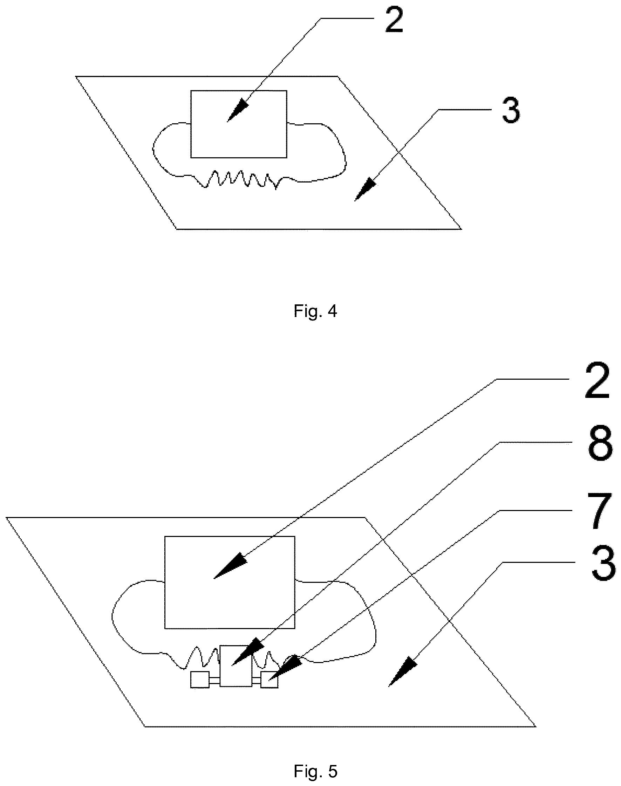

Please with reference to Fig. 4, in the embodiment of the present invention, an anti-fatigue driving massage device comprises a control system 1, a control device 2, a fixing device 3, a magnetic film 4, a power supply 5 and a man-machine interactive device 6, wherein the power supply 5 is a vehicular electric power supply; the control system 1 is respectively connected with the control device 2, the power supply 5 and the man-machine interactive device 6; the control device 2 is fixedly installed on the fixing device 3; the fixing device 3 is a sun shield; and the control device 2 is a loop current control device with a coil.

The usage method of the anti-fatigue driving massage device comprises the specific steps as follows:
1) connecting the control system 1 to the vehicular electric power supply, fixedly installing the loop current control device with the coil on the sun shield, and sticking the magnetic film 4 to the muscle around the eye of the driver, wherein the magnetic film 4 is required not to shield the sight line of the driver;
2) setting or regulating the massage strength and period through the man-machine interactive device 6;
3) obtaining the parameters of a current direction and a current size matched with the present massage strength and period by the control system 1 through corresponding algorithm processing; and
4) alternately controlling the polarity of the magnetic field around the loop current control device with the coil by the control system 1 through the parameters obtained in step 3), thereby controlling the magnetic film 4 to massage the muscle around the eye.

### Embodiment 3

Please with reference to Fig. 5, in the embodiment of the present invention, an anti-fatigue driving massage device comprises a control system 1, a control device 2, a fixing device 3, a magnetic film 4, a power supply 5 and a man-machine interactive device 6, wherein the power supply 5 is a battery; the control system 1 is respectively connected with the control device 2, the power supply 5 and the man-machine interactive device 6; the control device 2 is fixedly installed on the fixing device 3; the fixing device 3 is a sun shield; and the control device 2 is a control device with a motor 8 and magnets 7.

The usage method of the anti-fatigue driving massage device comprises the specific steps as follows:
1) installing the control system 1 and the battery, fixedly installing the control device with the motor 8 and the magnets 7 on the sun shield, and sticking the magnetic film 4 to the muscle around the eye of the driver, wherein the magnetic film 4 is required not to shield the sight line of the driver;
2) setting or regulating the massage strength and period through the man-machine interactive device 6;
3) obtaining the rotational speed of the motor 8 matched with the present massage strength and period by the control system 1 through corresponding algorithm processing; and
4) alternately controlling the polarity of the magnetic field around the control device with the motor 8 and the magnets 7 by the control system 1 through the rotational speed obtained in step 3), thereby controlling the magnetic film 4 to massage the muscle around the eye.

In the present invention, the control system 1 is used for automatically controlling the magnetic film 4 at regular time to perform active anti-fatigue massage, stimulus and the like for alerting the driver and relieving the fatigue of the driver, so that the driver keeps a conscious driving state; and the driver does not need to perform any operation during driving, thereby improving the driving safety.

The better embodiments of the present patent are described in detail above, but the present patent is not limited to the above-mentioned embodiments. Various modifications can be made by those skilled in the art within their knowledge scope.

## Claims

1. An anti-fatigue driving massage device comprising a control system (1), a control device (2) and a power supply (5), **characterized by** comprising a sun shield (3), a magnetic film (4), and a man-machine interactive device (6), wherein said control system (1) is respectively connected with the control device (2), the power supply (5) and the man-machine interactive device (6); said control device (2) is fixedly installed on the sun shield (3); said magnetic film (4) is stuck to the muscle around an eye of a driver; said control device (2) is a loop current control device with a coil; and said control system (1) is configured to obtain parameters of a current direction and a current size through corresponding algorithm processing and then alternately control a polarity of a magnetic field around the loop current control device with the coil.

2. The anti-fatigue driving massage device according to claim 1, **characterized in that** said power supply (5) is a battery or a vehicular electric power supply.

## Patentansprüche

1. Anti-Ermüdungs-Antriebsmassagevorrichtung mit einem Steuersystem (1), einer Steuervorrichtung (2) und einer Stromversorgung (5), **dadurch gekennzeichnet, dass** sie eine Sonnenblende (3), einen magnetischen Film (4) und eine Mensch-Maschine-Interaktionsvorrichtung (6) umfasst, wobei das Steuersystem (1) jeweils mit der Steuervorrichtung (2), der Stromversorgung (5) und der Mensch-Maschine-Interaktionsvorrichtung (6) verbunden ist; wobei die Steuervorrichtung (2) fest an der Sonnenblende (3) installiert ist; und wobei der magnetische Film (4) an dem Muskel um ein Auge eines Fahrers herum geklebt ist; wobei die Steuervorrichtung (2) eine Schleifenstromsteuervorrichtung mit einer Spule ist; und wobei das Steuersystem (1) konfiguriert ist, um Parameter einer Stromrichtung und einer Stromgröße durch entsprechende Algorithmusverarbeitung zu erhalten und abwechselnd eine Polarität eines Magnetfelds um die Schleifenstromsteuervorrichtung herum mit der Spule zu steuern.

2. Anti-Ermüdungs-Antriebsmassagevorrichtung nach Anspruch 1, **dadurch gekennzeichnet, dass** die Stromversorgung (5) eine Batterie oder eine elektrische Fahrzeugstromversorgung ist.

## Revendications

1. Dispositif de massage de conduite anti-fatigue comprenant un système de commande (1), un dispositif de commande (2) et une source d'énergie (5), **caractérisé en ce qu'**il comprend un écran solaire (3), un film magnétique (4) et un dispositif interactif homme-machine (6), dans lequel le système de commande (1) est respectivement connecté au dispositif de commande (2), à la source d'énergie (5) et au dispositif interactif homme-machine (6); le dispositif de commande (2) est installé de manière fixe sur l'écran solaire (3); le film magnétique (4) est collé au muscle autour d'un oeil d'un conducteur; le dispositif de commande (2) est une boucle de contrôle de courant avec une bobine; et le système de commande (1) est configuré pour obtenir des paramètres d'une direction de courant et une taille de courant par un traitement d'algorithme correspondant et ensuite contrôler alternativement une polarité d'un champ magnétique autour de la boucle de contrôle de courant avec la bobine.

2. Dispositif de massage de conduite anti-fatigue selon la revendication 1, **caractérisé en ce que** la source d'énergie (5) est une batterie ou une alimentation électrique de véhicule.
